# EUROPÄISCHE PATENTSCHRIFT

(11) **EP 2 596 775 B1**
(45) Veröffentlichungstag und Bekanntmachung des Hinweises auf die Patenterteilung: **10.06.2015**
(21) Anmeldenummer: 11009388.7
(22) Anmeldetag: 25.11.2011
(51) Int. Cl.: A61F 9/008

(54) **Vorrichtung zum Bearbeiten von Augengewebe mittels eines gepulsten Laserstrahls**
Device for treating eye tissue using a pulsed laser ray
Dispositif de traitement de tissu oculaire à l'aide d'un rayon laser pulsé

(43) Veröffentlichungstag der Anmeldung: 29.05.2013
(73) Patentinhaber: SIE AG, Surgical Instrument Engineering, 2562 Port (CH)
(72) Erfinder: Rathjen, Christian, 28197 Bremen (DE); Kanngiesser, Hartmut, 8055 Zuerich (CH); Ducry, Vincent, 1700 Fribourg (CH); Klauser, Lorenz, 3045 Meikirch (CH)
(74) Vertreter: Rentsch Partner AG

(56) Entgegenhaltungen:
- DE-A1-102005 013 949
- US-A1- 2011 028 958
- US-A1- 2011 196 350

## Beschreibung

### Technisches Gebiet

Die vorliegende Erfindung betrifft eine ophthalmologische Vorrichtung zum Bearbeiten von Augengewebe mittels eines gepulsten Laserstrahls. Die vorliegende Erfindung betrifft insbesondere eine ophthalmologische Vorrichtung mit einer Projektionsoptik zur fokussierten Projektion des Laserstrahls ins Augengewebe und ein der Projektionsoptik vorgelagertes Scannersystem zum strahlablenkenden Scannen des Augengewebes mit dem Laserstrahl.

### Stand der Technik

Zum Bearbeiten von Augengewebe mittels eines Laserstrahls wird ein Bearbeitungsbereich mit Laserpulsen gescannt (abgetastet), indem der gepulste Laserstrahl mittels geeigneter Scannersysteme (Ablenkvorrichtungen) in ein oder zwei Scannrichtungen (Abtastrichtungen) abgelenkt wird. Die Ablenkung der Lichtstrahlen respektive der Laserpulse, beispielsweise Femtosekundenlaserpulse, wird im Allgemeinen mit beweglichen Spiegeln vorgenommen, die um eine oder zwei Scannachsen schwenkbar sind, beispielsweise mit Galvanoscannern, Piezoscannern, Polygonscannern oder Resonanzscannern.

In US 7,621,637 wird eine Vorrichtung zum Bearbeiten von Augengewebe beschrieben, welche eine Basisstation mit einer Laserquelle zur Erzeugung von Laserpulsen und einen in der Basisstation angeordneten Scanner mit beweglichen Ablenkspiegeln zum Ablenken der Laserpulse in einer Scannrichtung aufweist. Die abgelenkten Laserpulse werden über ein optisches Übertragungssystem von der Basisstation zu einem Applikationskopf übertragen, der mittels einer mechanisch bewegten Projektionsoptik einen Arbeitsbereich gemäss einem Scannmuster abfährt. Die im Vergleich zur mechanischen Bewegung viel schnellere Ablenkung in der Scannrichtung wird im Applikationskopf auf die mechanische Bewegung der Projektionsoptik und somit auf dessen Scannmuster überlagert. Ein Fast-Scannersystem in der Basisstation ermöglicht eine feine Bewegung der Laserpulse (Microscann), welche auf das Scannmuster der beweglichen Projektionsoptik überlagert wird, die einen grossen Bearbeitungsbereich abdeckt, beispielsweise das gesamte Auge.

Die bekannten Systeme ermöglichen zwar die Bearbeitung von einfachen Scannmustern, beispielsweise das Schneiden eines Gewebelappens (Flap), das in der Regel als grosses Flächenstück mit einfacher Randgeometrie ausgeführt wird. Bei Anwendungen, in denen nicht nur Gewebeschnitte in einer im Wesentlichen horizontal ausgerichteten Bearbeitungsfläche auf einer gemeinsamen Fokalfläche ausgeführt werden, sondern in denen auch Schnitte mit einer vertikalen Schnittkomponente über unterschiedliche Fokushöhen ausgeführt werden sollen, z.B. schräg zur Horizontalen verlaufende oder vertikale Schnitte, erweist sich das vertikale Verfahren der Projektionsoptik oder wenigstens Teilen davon für eine vertikale Veränderung des Fokus und damit der Schnitthöhe als zu langsam um Schnitte mit einer vertikalen Komponente, also mit veränderlicher Fokustiefe während des Schneidens, auszuführen.

US 2011/196350 beschreibt ein Lasersystem für die Kataraktchirurgie mit zwei Laserquellen zur Erzeugung von gepulsten Laserstrahlen und einem Scannersystem. Die Laserstrahlen werden über eine z-Scann-Vorrichtung geleitet, welche die Fokusposition im Auge des Patienten in z-Richtung verschiebt. Die z-Scann-Vorrichtung kann automatisch und dynamisch gesteuert werden und unabhängig oder in Interaktion mit einer nachgeschalteten xy-Scann-Vorrichtung betrieben wird.

DE 10 2005 013949 beschreibt eine Scannvorrichtung zur Fokussierung eines Strahlenbündels, in welcher ein optisches Element vorgesehen ist, das in Abhängigkeit von der gewünschten Lage des Fokus in Richtung des Strahlenbündels bewegt wird. Durch die Bewegung des optischen Elements ist die Divergenz des Strahlenbündels veränderbar, so dass das Strahlenbündel ohne Bewegung der Fokussieroptik in verschiedenen Tiefen fokussierbar ist.

US 2011/028958 beschreibt ein ophthalmologisches Lasersystem mit einem xy-Scanner und einem der Projektionsoptik vorgelagerten z-Scanner. Gemäss US 2011/028958 muss zur Erzeugung eines geraden Transversalschnitts die Fokustiefe kontinuierlich und synchron mit dem xy-Scanner nachgestellt werden, um die Krümmung der Fokalfläche zu kompensieren.

### Darstellung der Erfindung

Es ist eine Aufgabe der vorliegenden Erfindung eine Vorrichtung zum Bearbeiten von Augengewebe mittels eines gepulsten Laserstrahls vorzuschlagen, welche zumindest einige Nachteile des Stands der Technik nicht aufweist. Es ist insbesondere eine Aufgabe der vorliegenden Erfindung eine Vorrichtung zum Bearbeiten von Augengewebe mittels eines gepulsten und durch eine Projektionsoptik fokussierten Laserstrahls vorzuschlagen, welche Gewebeschnitte mit einer vertikalen Schnittkomponente ermöglicht, ohne dazu vertikale Verschiebungen der Projektionsoptik vornehmen zu müssen.

Gemäss der vorliegenden Erfindung werden diese Ziele durch die Merkmale der unabhängigen Ansprüche erreicht. Weitere vorteilhafte Ausführungsformen gehen ausserdem aus den abhängigen Ansprüchen und der Beschreibung hervor.

Eine ophthalmologische Vorrichtung zum Bearbeiten von Augengewebe mittels eines gepulsten Laserstrahls umfasst eine Projektionsoptik zur fokussierten Projektion des Laserstrahls respektive der Laserpulse ins Augengewebe und ein der Projektionsoptik vorgelagertes Scannersystem zum strahlablenkenden Scannen des Augengewebes mit dem Laserstrahl respektive den Laserpulsen in einer über einen Scannwinkel ausgeführten Scannbewegung entlang einer Scannlinie.

Die oben genannten Ziele werden durch die vorliegende Erfindung insbesondere dadurch erreicht, dass dem Scannersystem ein Divergenzmodulator zum dynamischen Verändern der Divergenz des Laserstrahls vorgelagert ist. Die dynamische Veränderung der Divergenz des Laserstrahls während der Scannbewegung bewirkt eine sich abhängig von der Scannbewegung verändernde Divergenz des Laserstrahls. Dies entspricht einer Modulation der Divergenz des Laserstrahls in einer zur optischen Übertragungsachse senkrecht verlaufenden Richtung.

Der Divergenzmodulator ist, zur Synchronisation von Scannbewegung und Divergenz des Laserstrahls, mit dem Scannersystem gekoppelt.

In einer Ausführungsvariante ist der Divergenzmodulator eingerichtet, die Divergenz des Laserstrahls mit mindestens gleich grosser Frequenz zu modulieren, mit der das Scannersystem die Scannbewegung ausführt.

In einer Ausführungsvariante ist das Scannersystem eingerichtet, die Scannbewegung über einen Scannwinkel auszuführen und der Divergenzmodulator ist eingerichtet, die Divergenz des Laserstrahls für eine vom Scannwinkel abhängige Verschiebung der fokussierten Projektion in Projektionsrichtung zu modulieren.

In einer weiteren Ausführungsvariante ist der Divergenzmodulator eingerichtet, die Divergenz des Laserstrahls für eine gezielte Verkippung der Scannlinie zu modulieren.

Die Verkippung der Scannlinie ermöglicht eine von der Scannbewegung respektive vom Scannwinkel abhängige Verschiebung des Fokus der ins Augengewebe projizierten Laserpulse ohne vertikale Verschiebung der Projektionsoptik.

In einer Ausführungsvariante ist der Divergenzmodulator eingerichtet, die Divergenz des Laserstrahls für eine Verkippung der Scannlinie mit einem definierten Verkippungswinkel in einer durch die Scannlinie und eine optische Achse der Projektionsoptik definierten Ebene zu modulieren.

In einer Ausführungsvariante ist der Divergenzmodulator eingerichtet, die Divergenz des Laserstrahls mit grösserer Frequenz zu modulieren, als das Scannersystem die Scannbewegung ausführt, und die Divergenz des Laserstrahls für eine gezielte Verformung der Scannlinie zu modulieren.

In einer weiteren Ausführungsvariante umfasst die ophthalmologische Vorrichtung ein dem Scannersystem nachgelagertes weiteres, zweites Scannersystem zum Scannen des Augengewebes mit dem Laserstrahl entlang einer Bearbeitungslinie, wobei die Scannbewegung des vorgelagerten, ersten Scannersystems der Bearbeitungslinie überlagert ist, und wobei der Divergenzmodulator eingerichtet ist, die Divergenz des Laserstrahls für eine gezielte Verkippung einer durch die Scannlinie und die Bearbeitungslinie definierten Schneidfläche zu modulieren.

Das erste Scannersystem ist beispielsweise eingerichtet, die Scannbewegung mit einer wesentlich höheren Geschwindigkeit auszuführen, als das zweite Scannersystem das Augengewebe entlang der Bearbeitungslinie scannt.

In einer Ausführungsvariante umfasst die ophthalmologische Vorrichtung ein dem Scannersystem nachgelagertes weiteres, zweites Scannersystem zum Scannen des Augengewebes mit dem Laserstrahl entlang einer Bearbeitungslinie, wobei die Scannbewegung des vorgelagerten, ersten Scannersystems der Bearbeitungslinie überlagert ist, und der Divergenzmodulator ist eingerichtet, die Divergenz des Laserstrahls mit grösserer Frequenz zu modulieren, als das erste Scannersystem die Scannbewegung ausführt, und die Divergenz des Laserstrahls für eine gezielte Verformung einer durch die Scannlinie und die Bearbeitungslinie definierten Schneidfläche zu modulieren.

In der Erfindung umfasst die ophthalmologische Vorrichtung ein Steuermodul, das eingerichtet ist, den Divergenzmodulator derart zu steuern, dass die Divergenz des Laserstrahls so moduliert wird, dass die Scannlinie mit einem vorgegebenen Verkippungswinkel verkippt wird.

In einer Ausführungsvariante ist das Steuermodul eingerichtet, den Divergenzmodulator derart zu steuern, dass die Divergenz des Laserstrahls so moduliert wird, dass die Scannlinie in einer durch die Scannlinie und eine optische Achse der Projektionsoptik definierten Ebene mit dem vorgegebenen Verkippungswinkel verkippt wird.

In einer weiteren Ausführungsvariante ist das Steuermodul eingerichtet, während des Bearbeitens des Augengewebes einen veränderten Verkippungswinkel zu bestimmen und den Divergenzmodulator derart zu steuern, dass die Scannlinie mit dem veränderten Verkippungswinkel verkippt wird.

In einer weiteren Ausführungsvariante umfasst der Divergenzmodulator zwei seriell angeordnete optische Linsen, wobei mindestens eine der Linsen zur Modulation der Divergenz des Laserstrahls, auf einer optischen Übertragungsachse verschiebbar, mit einem Bewegungstreiber gekoppelt ist.

In alternativen Ausführungsvarianten umfasst der Divergenzmodulator einen räumlichen Lichtmodulator zum Modulieren der Wellenfront des Laserstrahls, einen Flächenlichtmodulator zum Modulieren der Reflexionswinkel an mehreren Punkten einer Reflexionsfläche, einen Brechungsmodulator zum Modulieren des Brechungsindexes eines optischen Elements an mehreren Punkten im Strahlengangquerschnitt und/oder einen Amplitudenmodulator zur Amplitudenmodulation an mehreren Punkten im Strahlengangquerschnitt des Laserstrahls.

In einer weiteren Ausführungsvariante ist die Projektionsoptik mit einer Fokussiervorrichtung zum Einstellen einer Fokustiefe versehen.

### Kurze Beschreibung der Zeichnungen

Nachfolgend wird eine Ausführung der vorliegenden Erfindung anhand eines Beispiels beschrieben. Das Beispiel der Ausführung wird durch die folgenden beigelegten Figuren illustriert:
- Figur 1:: zeigt ein Blockdiagram, das schematisch eine ophthalmologische Vorrichtung zur Bearbeitung von Augengewebe mit einem gepulsten Laserstrahl illustriert, welche ein Scannersystem zum Abtasten des Augengewebes entlang einer Scannlinie und ein Verkippungssystem zum Verkippen der Scannlinie umfasst.
- Figur 1a:: zeigt ein Blockdiagram der ophthalmologischen Vorrichtung, in welcher zum Verkippen der Scannlinie der Projektionsoptik mindestens ein optisches Element vorgelagert ist, das im Strahlengang eine vom Scannwinkel abhängige Divergenz des Laserstrahls erzeugt.
- Figur 1b:: zeigt ein Blockdiagram der ophthalmologischen Vorrichtung, in welcher zum Verkippen der Scannlinie dem Scannersystem ein Divergenzmodulator vorgelagert ist, der die Divergenz des Laserstrahls dynamisch ändert.
- Figur 1c:: zeigt ein Blockdiagram eines Applikationskopfs der ophthalmologischen Vorrichtung, in welchem die Projektionsoptik zum Verkippen der Scannlinie um eine Drehachse verkippbar ist.
- Figur 1d:: zeigt ein Blockdiagram, in welchem die Projektionsoptik und entsprechend die Scannlinie in einem verkippten Zustand illustriert sind.
- Figur 2a:: zeigt einen schematischen Querschnitt durch einen Abschnitt des Strahlengangs, welcher die Scannbewegung des Laserstrahls um einen Scannwinkel und die dadurch bewirkte Bewegung des Fokus des Lasterstrahls entlang der Scannlinie illustriert.
- Figur 2b:: zeigt den schematischen Querschnitt des Strahlengangabschnitts bei einer Veränderung der Divergenz des Laserstrahls abhängig vom Scannwinkel und die dadurch bewirkte Verkippung der Scannlinie um einen Verkippungswinkel.
- Figur 3:: zeigt einen schematischen Querschnitt eines Abschnitt des Strahlengangs in einem Divergenzmodulator mit mindestens einer verschiebbaren Linse und illustriert die durch die Verschiebung der Linse veränderte Divergenz des Laserstrahls.
- Figur 4a:: zeigt in der Aufsicht auf die Cornea die Überlagerung der Scannbewegung auf eine Bearbeitungslinie zur Bearbeitung des Augengewebes in einem ausgedehnten Bearbeitungsgebiet.
- Figur 4b:: zeigt im Querschnitt der Cornea die verkippte Scannlinie entlang der das Augengewebe durch den gepulsten Laserstrahl abgetastet und bearbeitet wird.
- Figur 4c:: zeigt im Querschnitt der Cornea die verkippten Scannlinien mehrerer Bearbeitungsbahnen entlang denen das Augengewebe durch den gepulsten Laserstrahl abgetastet und bearbeitet wird.
- Figur 5:: zeigt im oberen Teil in der Aufsicht der Cornea ein Anwendungsbeispiel eines kreisbogenförmigen Kegelbahnschnitts, der, wie im unteren Teil dargestellten Querschnitt der Cornea illustriert ist, auf einer verkippten Scannlinie basiert, die entlang einer kreisbogenförmigen Bearbeitungslinie verfahren wird.
- Figur 6:: zeigt im oberen Teil in der Aufsicht der Cornea ein Anwendungsbeispiel von zwei kreisbogenförmigen Vertikalschnitten, die, wie im unteren Teil dargestellten Querschnitt der Cornea illustriert ist, jeweils auf einer verkippten Scannlinie basieren, die in der Richtung einer kreisbogenförmigen Bearbeitungslinie ausgerichtet, entlang der betreffenden Bearbeitungslinie verfahren wird.
- Figur 7:: zeigt im oberen Teil in der Aufsicht der Cornea ein Anwendungsbeispiel von mehreren auf ein gemeinsames Zentrum ausgerichteten Vertikalschnitten, die, wie im unteren Teil dargestellten Querschnitt der Cornea illustriert ist, jeweils auf einer verkippten Scannlinie basieren, die in der Richtung einer aufs Zentrum ausgerichteten Bearbeitungslinie ausgerichtet, entlang der betreffenden Bearbeitungslinie verfahren wird.

### Wege zur Ausführung der Erfindung

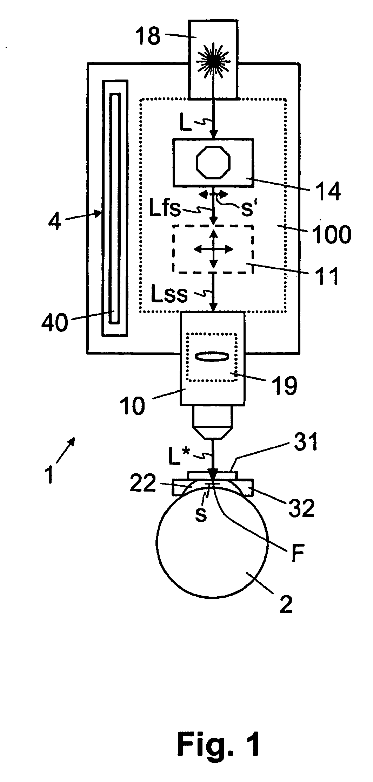

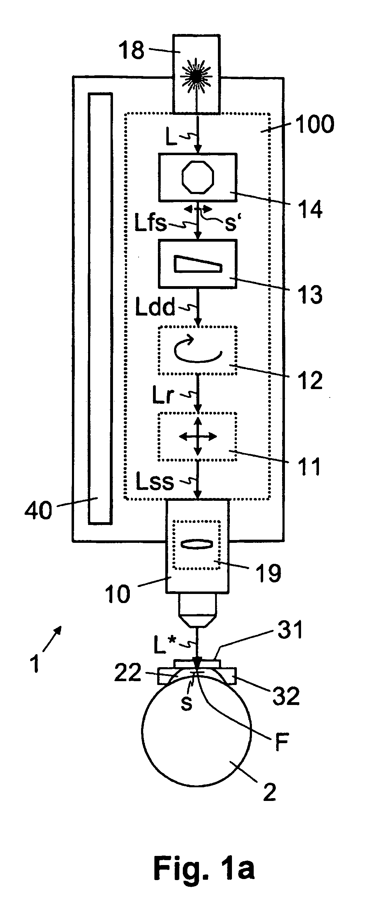

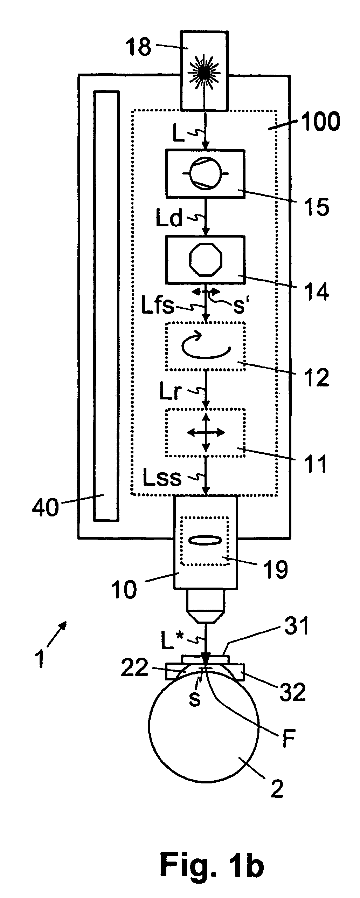

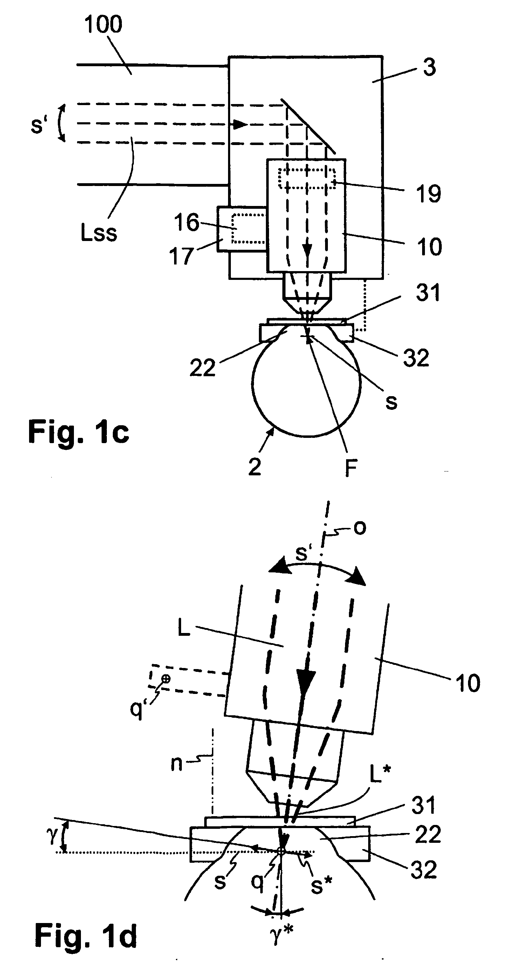

In den Figuren 1, 1a und 1b bezieht sich das Bezugszeichen 1 jeweils auf eine ophthalmologische Vorrichtung zum Bearbeiten von Augengewebe mittels Laserpulsen, beispielsweise die Cornea 22 oder anderes Gewebe eines Auges 2.

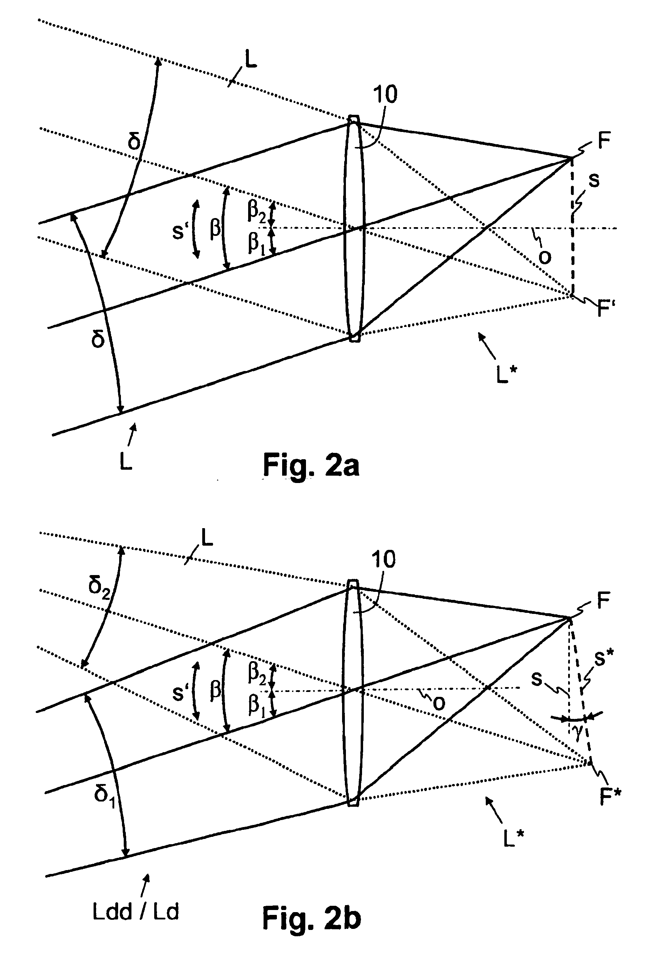

Wie in den Figuren 1, 1a und 1b schematisch dargestellt ist, umfasst die ophthalmologische Vorrichtung 1 ein optisches Übertragungssystem 100 zur Übertragung von Laserpulsen eines von einer Laserquelle 18 gelieferten gepulsten Laserstrahls L zu einer Projektionsoptik 10. Die Projektionsoptik 10 ist eingerichtet zur fokussierten Projektion des gepulsten Laserstrahls L respektive der Laserpulse für die punktuelle Gewebeauflösung in einem Fokus F (Brennpunkt) im Innern des Augengewebes. In den Figuren 1, 1a, 1b, 1d, 2a und 2b wird der von der Projektionsoptik 10 projizierte Laserstrahl L mit dem Bezugszeichen L* bezeichnet.

Die Laserquelle 18 umfasst insbesondere einen Femtosekundenlaser zur Erzeugung von Femtosekundenlaserpulsen, die Pulsbreiten von typisch 10fs bis 1000fs (1fs=10⁻¹⁵s) aufweisen. Die Laserquelle 18 ist in einem separaten oder in einem mit der Projektionsoptik 10 gemeinsamen Gehäuse angeordnet.

An dieser Stelle soll festgehalten werden, dass mit dem Bezugszeichen L allgemein der gepulste Laserstrahl L respektive dessen Laserpulse im Strahlengang von der Laserquelle 18 bis zum Fokus F bezeichnet werden, dass jedoch abhängig vom Zusammenhang auch weitere Bezugszeichen verwendet werden, um den gepulsten Laserstrahl L respektive dessen Laserpulse an einer bestimmten Stelle im Strahlengang respektive im optischen Übertragungssystem 100 zu bezeichnen.

Wie in der Figur 1c dargestellt ist, ist die Projektionsoptik 10 beispielsweise in einen Applikationskopf 3 eingebaut, der auf das Auge 2 aufsetzbar ist. Der Applikationskopf 3 wird vorzugsweise über einen mindestens stellenweise lichtdurchlässigen Kontaktkörper 31 auf das Auge 2 aufgesetzt und beispielsweise mittels eines vakuumgesteuerten Saugrings 32 am Auge 2 fixiert, wobei der Kontaktkörper 31 und der Saugring 32 fest oder entfernbar mit dem Applikationskopf 3 verbunden sind. Die Projektionsoptik 10 umfasst in einer Ausführungsvariante eine Fokussiervorrichtung 19 zum Einstellen der Fokustiefe, beispielsweise eine oder mehrere bewegliche Linsen oder einen Antrieb zum Bewegen der gesamten Projektionsoptik 10.

Wie in den Figuren 1, 1a und 1b ersichtlich ist, umfasst die ophthalmologische Vorrichtung 1 mindestens ein der Projektionsoptik 10 vorgelagertes Scannersystem 14 zum Abtasten des Augengewebes entlang einer Scannlinie s. Das Scannersystem 14 ist eingerichtet, den gepulsten Laserstrahl L respektive die Laserpulse abzulenken um das Augengewebe bearbeitend zu scannen (abzutasten). Das Scannersystem 14 umfasst einen oder mehrere bewegliche Ablenkspiegel, beispielsweise ein rotierender Polygonspiegel (Polygonscanner), und ermöglicht ein strahlablenkendes Scannen des Augengewebes mit dem gepulsten Laserstrahl L in einer über einen Scannwinkel β ausgeführten Scannbewegung s' entlang einer Scannlinie s, wie in der Figur 2a illustriert ist. Durch die Scannbewegung s' wird der Fokus F des projizierten Laserstrahls L* entlang der Scannlinie s bewegt, beispielsweise ausgehend von der Position des Fokus F bei einer Ablenkung des Laserstrahls L mit dem Scannwinkel β₁ zur Position des Fokus F' bei einer Ablenkung des Laserstrahls L mit dem Scannwinkel β₂.

Das strahlablenkende Scannersystem 14 ist abhängig von Betriebsmodus und/oder Konstruktion als resonanter, oszillierender, oder frei adressierbarer Scanner ausgeführt und umfasst beispielsweise einen Galvanoscanner, einen Piezo angetriebenen Scanner, einen MEM (Micro Electro-Mechanical) Scanner, einen AOM (Acusto-optische Modulatoren) Scanner oder einen EOM (Electro-optische Modulatoren) Scanner.

Wie in den Figuren 1 a und 1 b dargestellt ist, umfasst die ophthalmologische Vorrichtung 1 in einer Ausführungsvariante ein im Strahlengang angeordnetes, dem Scannersystem 14 nachgeschaltetes Rotationselement 12 zum Drehen einer durch die Scannbewegung s' und die optische Übertragungsachse definierten Scannebene um die optische Übertragungsachse, beispielsweise ein K-Spiegel. In den Figuren 1a und 1b wird der Laserstrahl L mit der vom Rotationselement 12 gedrehten Scannebene mit dem Bezugszeichen Lr bezeichnet.

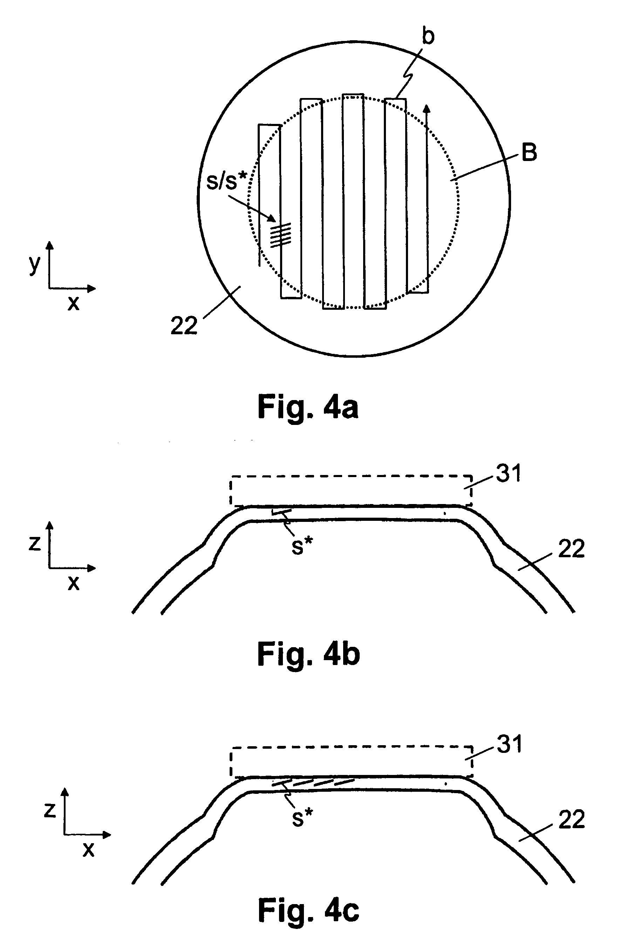

In einer Ausführungsvariante umfasst die ophthalmologische Vorrichtung 1 ein weiteres optionales Scannersystem 11, das der Projektionsoptik 10 vorgelagert ist und dem Scannersystem 14 nachgeschaltet ist. Das Scannersystem 11 ist eingerichtet, das Augengewebe mit dem gepulsten Laserstrahl L respektive den Laserpulsen entlang einer Bearbeitungslinie b zu scannen, wie beispielhaft in der Aufsicht der Figur 4a illustriert ist. Im Beispiel der Figur 4a ist eine mäandrierende Bearbeitungslinie b dargestellt, der Fachmann wird verstehen, dass die Bearbeitungslinie b ja nach Anwendung und Ansteuerung des Scannersystems 11 auch andere Linienformen annehmen kann, beispielsweise spiralförmig, kreisförmig oder eine freie Form, die sich über einen Teil oder das gesamte zu bearbeitende Gebiet B des Augengewebes erstreckt. Das Scannersystem 11 ist als mechanischer Scanner ausgeführt, der die Projektionsoptik 10 mittels einem oder mehreren Bewegungstreibern über das gesamte Bearbeitungsgebiet B entlang der Bearbeitungslinie b abfährt, oder das Scannersystem 11 ist strahlablenkend ausgeführt, beispielsweise als Galvanoscanner, und umfasst einen oder zwei um jeweils eine oder zwei Achsen bewegliche Ablenkspiegel zum Ablenken des gepulsten Laserstrahls L respektive der Laserpulse über das gesamte Bearbeitungsgebiet B entlang der Bearbeitungslinie b.

Das dem Scannersystem 11 vorgelagerte Scannersystem 14 weist eine Scanngeschwindigkeit auf, die ein Vielfaches der Scanngeschwindigkeit des Scannersystems 11 beträgt. Entsprechend kann das Scannersystem 14 auch als Fast-Scan-System bezeichnet, das den abgelenkten Laserstrahl Lfs erzeugt, und das Scannersystem 11 kann als Slow-Scan-System bezeichnet werden, das den abgelenkten Laserstrahl Lss erzeugt. Die beiden Scannersysteme 11, 14 sind so eingerichtet und gekoppelt, dass die entlang der Scannlinie s verlaufende Scannbewegung s' der Bearbeitungslinie b überlagert ist, wie in der x/y-Aufsicht der Figur 4a schematisch und beispielhaft dargestellt ist. Wie dje Scannlinie s bei einem einzigen Scannersystem 14 mit einer einzigen Scannachse eine Scheidlinie definiert so wird durch die Überlagerung der Scannlinie s auf die Bearbeitungslinie b eine Schneidfläche definiert.

Wie in der Figur 1 schematisch dargestellt ist, umfasst die ophthalmologische Vorrichtung 1 überdies ein Verkippungssystem 4 zum Verkippen der Scannlinie s respektive der Schneidfläche. Wie in den Figuren 1d und 2b ersichtlich ist, ist die verkippte Scannlinie s* gegenüber der Scannlinie s in einer durch die optische Achse o der Projektionsoptik 10 und der Scannlinie s verlaufenden Ebene um den Verkippungswinkel γ verkippt. Entsprechend ermöglicht das Verkippungssystem 4 auch eine Verkippung der durch die Scannlinie s und Bearbeitungslinie b definierten Schneidfläche um den Verkippungswinkel γ, beispielsweise je nach Anwendung und Steuerung eine Verkippung der gesamten Schneidfläche oder einzelner durch die Bearbeitungslinie b definierten Bahnen.

Im Beispiel der Figur 4a ist die entlang der Scannlinie s respektive der verkippten Scannlinie s* verlaufende Scannbewegung s' der mäandrierenden Bearbeitungslinie b überlagert und erzeugt so beim Abfahren der gesamten Bearbeitungslinie b eine über das Bearbeitungsgebiet B ausgedehnte Schneidfläche. Die Figuren 4b und 4c zeigen die der Figur 4a entsprechenden Querschnittsdarstellungen der mittels des Kontaktkörpers 31 applanierten Cornea 22 nachdem eine Bahn respektive vier aufeinanderfolgende Bahnen der Bearbeitungslinie b mit einer Scannbewegung s' entlang der verkippten Scannlinie s* abgefahren wurden. Die verkippte Scannlinie s* des in den Figuren 4a, 4b, 4c dargestellten Anwendungsbeispiels ermöglicht die Korrektur respektive Entfernung von Restfehlern bei aneinandergrenzenden Bearbeitungsbahnen, die dann entstehen, wenn die optische Achse o der Projektionsoptik 10 nicht normal zur Applanationsfläche des Kontaktkörpers 31 ausgerichtet ist.

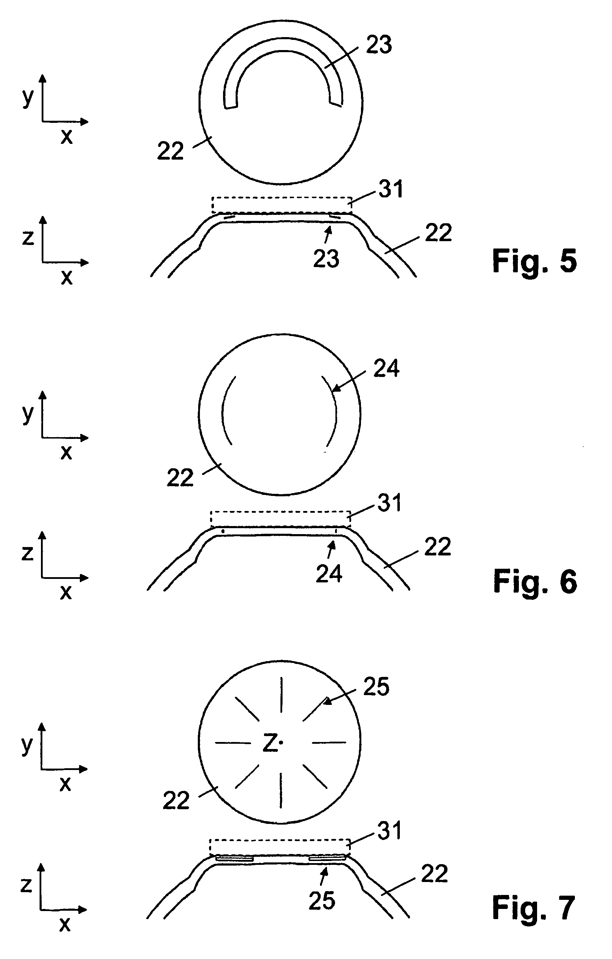

Das Anwendungsbeispiel der Figur 5 zeigt im oberen Teil eine Aufsicht auf die Cornea 22 und einen darin ausgeführten, schematisch dargestellten Kegelbahnschnitt 23, dessen Querschnitt im unteren Teil der Figur 5 illustriert ist. Der Kegelbahnschnitt 23 wird durch die Überlagerung einer entlang einer verkippten Scannlinie s* ausgeführten Scannbewegung s' auf eine kreisbogenförmige Bearbeitungslinie b erzeugt, wobei die verkippte Scannlinie s* beispielsweise senkrecht zur Bearbeitungslinie b ausgerichtet ist.

Das Anwendungsbeispiel der Figur 6 zeigt im oberen Teil eine Aufsicht auf die Cornea 22 und zwei darin ausgeführte, schematisch dargestellte kreisbogenförmige Vertikalschnitte 24, von denen ein Querschnitt im unteren Teil der Figur 6 illustriert ist. Die beiden Vertikalschnitte 24 werden jeweils durch die Überlagerung einer entlang einer verkippten Scannlinie s* ausgeführten Scannbewegung s' auf eine kreisbogenförmige Bearbeitungslinie b erzeugt, wobei die verkippte Scannlinie s* zudem in der Richtung der betreffenden Bearbeitungslinie b ausgerichtet ist.

Das Anwendungsbeispiel der Figur 7 zeigt im oberen Teil eine Aufsicht auf die Cornea 22 und mehrere darin ausgeführte, schematisch dargestellte Vertikalschnitte 25, die geradlinig auf einen Zentrumspunkt Z (Vertex) der Cornea 22 ausgerichtet sind und von denen ein Querschnitt im unteren Teil der Figur 6 illustriert ist. Die Vertikalschnitte 25 werden jeweils durch die Überlagerung einer entlang einer verkippten Scannlinie s* ausgeführten Scannbewegung s' auf eine gradlinige, auf den Zentrumspunkt Z (Vertex) der Cornea 22 ausgerichtete Bearbeitungslinie b erzeugt, wobei die verkippte Scannlinie s* zudem jeweils in der Richtung der betreffenden Bearbeitungslinie b ausgerichtet ist. Zur Steuerung der Verkippung der Scannlinie s respektive der Schneidfläche umfasst das Verkippungssystem 4 ein Steuermodul 40, welches eingerichtet ist, Komponenten des Verkippungssystems 4 derart zu steuern, dass die Scannlinie s (und damit ggf. auch die Schneidfläche) in einer durch die optische Achse o der Projektionsoptik 10 und der Scannlinie s verlaufenden Ebene um einen vorgegebenen Verkippungswinkel γ verkippt wird. Der Verkippungswinkel γ ist beispielsweise fest definiert, wird über eine Benutzerschnittstelle eingegeben oder wird durch eine Steuerungsfunktion des Steuermoduls 40 während der Bearbeitung des Augengewebes laufend verändert. Das Steuermodul 40 umfasst eine programmierbare Steuervorrichtung, beispielsweise ein oder mehrere Prozessoren mit Programm- und Datenspeicher sowie programmierte Softwaremodule zur Steuerung der Prozessoren.

Abhängig von der Ausführungsvariante umfasst das Verkippungssystem 4 unterschiedliche Komponenten, die zur Verkippung der Scannlinie s vorgesehen und zur Steuerung mit dem Steuermodul 40 verbunden sind.

Die Figur 1a stellt eine ophthalmologische Vorrichtung 1 dar, in welcher das Verkippungssystem 4 eines oder mehrere optische Elemente 13 umfasst, die der Projektionsoptik 10 vorgelagert im Strahlengang vom Scannersystem 14 zur Projektionsoptik 10 angeordnet sind, und die eingerichtet sind im Strahlengang eine vom Scannwinkel β, β₁, β₂ abhängige Divergenz δ, δ₁, δ₂ des Laserstrahls L zu erzeugen (siehe Figur 2b).

Wie in der Figur 1a dargestellt ist, ist das optische Elemente 13 dem Scannersystem 14 nachgelagert und verändert die Divergenz δ, δ₁, δ₂ des Laserstrahls L abhängig vom Scannwinkel β, β₁, β₂ des vom Scannersystems 14 abgelenkten Laserstrahls L. Der Laserstrahl L mit der vom optischen Element 13 abhängig vom Scannwinkel β, β₁, β₂ veränderten Divergenz δ, δ₁, δ₂ wird in den Figuren 1 a und 2b mit dem Bezugszeichen Ldd bezeichnet. In der Ausführungsvariante mit dem optionalen Rotationselement 12 sind die optischen Elemente 13 respektive das optische Element 13 im Strahlengang dem Rotationselement 12 vorgeschaltet.

Wie in der Figur 2a dargestellt ist, weist der Laserstrahl L ohne Präsenz des optischen Elements 13 bei verschiedenen Scannwinkeln β, β₁, β₂, jeweils eine unveränderte, konstante Divergenz δ auf. Oft wird eine Divergenz 8 von annähernd Null eingestellt. Bei vorhandenem optischem Element 13 weist der Laserstrahl L jedoch wie in der Figur 2b illustriert bei verschiedenen Scannwinkeln β, β₁, β₂ eine unterschiedliche Divergenz δ₁, δ₂ auf. Wie in der Figur 2b ersichtlich ist, ist das optische Element 13 so eingerichtet, dass es die Divergenz δ, δ₁, δ₂ des Laserstrahls L abhängig vom Scannwinkel β, β₁, β₂ so verändert, dass sich der Fokus F, F* des projizierten Laserstrahls abhängig vom Scannwinkel β, β₁, β₂ in der Projektionsrichtung verschiebt und sich eine verkippte Scannlinie s* ergibt, die in der durch die optische Achse o der Projektionsoptik 10 und der Scannlinie S gebildeten Ebene um den Verkippungswinkel γ von der unverkippten Scannlinie s verkippt ist. Bei einer Verkippung der Scannlinie s wird das Augengewebe durch das Scannersystem 14 mit dem gepulsten Laserstrahl L in einer über den Scannwinkel β ausgeführten Scannbewegung s' strahlablenkend entlang der verkippten Scannlinie s* gescannt, wie in der Figur 2b illustriert ist. Dabei wird der Fokus F des projizierten Laserstrahls L* durch die Scannbewegung s' entlang der verkippten Scannlinie s* bewegt, beispielsweise ausgehend von der Position des Fokus F bei einer Ablenkung des Laserstrahls L mit dem Scannwinkel β₁ hin zur Position des Fokus F* bei einer Ablenkung des Laserstrahls L mit dem Scannwinkel β₂.

Ausführungen der optischen Elemente 13 respektive des optischen Elements 13 umfassen beispielsweise Keilplatten, Prismen, Linsen, diffraktive optische Elemente und asphärische Spiegel.

In einer alternativen Ausführungsvariante ist das optische Element 13 direkt im Scannersystem 14 angeordnet; und beispielsweise als Ablenkspiegel ausgestaltet, der eine veränderliche Oberflächenkrümmung aufweist.

Zum Einstellen der vom Scannwinkel β, β₁, β₂ abhängigen Divergenz δ, δ₁, δ₂ des Laserstrahls L sind die optischen Elemente 13 respektive das optische Element 13 in den Strahlengang einschiebbar respektive aus dem Strahlengang hinausschiebbar. Als Alternative oder zusätzlich sind die optischen Elemente 13 respektive das optische Element 13 zum Einstellen der vom Scannwinkel β, β₁, β₂ abhängigen Divergenz δ, δ₁, δ₂ des Laserstrahls L einstellbar respektive justierbar, beispielsweise durch Rotation der optischen Elemente 13 um die optische Achse o, durch Verkippung der optischen Elemente 13 um eine Drehachse oder durch Verschiebung der optischen Elemente 13 entlang einer zur optischen Achse o verkippten Translationsachse.

In der Ausführungsvariante mit dem optionalen Scannersystem 11, welches das Augengewebe mit dem Laserstrahl L respektive den Laserpulsen entlang einer Bearbeitungslinie b scannt, der die Scannbewegung s' des vorgeschalteten Scannersystems 14 überlagert ist, sind die optischen Elemente 13 eingerichtet, die vom Scannwinkel β, β₁, β₂ abhängige Divergenz δ, δ₁, δ₂ des Laserstrahls L für eine gezielte Verkippung einer durch die Scannlinie s und die Bearbeitungslinie b definierten Schneidfläche zu erzeugen.

Das Steuermodul 40 ist eingerichtet die optischen Elemente 13 respektive das optische Element 13 so einzustellen, dass durch die vom Scannwinkel β, β₁, β₂ abhängige Divergenz δ, δ₁, δ₂ des Laserstrahls L eine Verkippung der Scannlinie s respektive der durch die Scannlinie s und die Bearbeitungslinie b definierten Schneidfläche um den vorgegebenen Verkippungswinkel γ bewirkt wird. Dazu umfasst das Steuermodul 40 eine Steuerfunktion, welche für vorgegebene respektive während der Bearbeitung des Augengewebes laufend neu berechnete Verkippungswinkel γ jeweils zugeordnete Steuerwerte zur Einstellung der optischen Elmente 13 bestimmt, beispielsweise Steuerwerte zum Einstellen eines Rotationswinkels der optischen Elemente 13 um die optische Achse o, eines Verkippungsgrads der optischen Elemente 13 um eine Drehachse oder einer Position der optischen Elemente 13 auf einer zur optischen Achse o verkippten Translationsachse, wodurch die relative Lage der optischen Elemente 13 im Strahlquerschnitt definiert ist, oder einer Oberflächenkrümmung der optischen Elemente 13.

Die Figur 1b stellt eine ophthalmologische Vorrichtung 1 dar, in welcher das Verkippungssystem 4 einen Divergenzmodulator 15 umfasst, der dem Scannersystem 14 vorgeschaltet ist, und der eingerichtet ist, die Divergenz δ des Laserstrahls L dynamisch zu verändern.

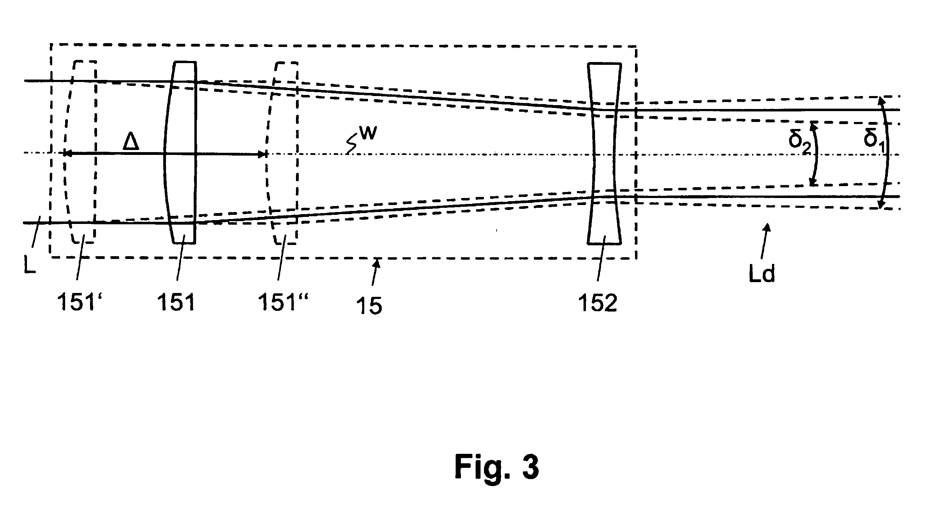

Die Figur 3 illustriert schematisch eine Ausführungsvariante des Divergenzmodulators 15 mit zwei seriell angeordneten optischen Linsen 151, 152, von denen mindestens eine zur Modulation der Divergenz 8 des Laserstrahls L auf einer optischen Übertragungsachse w verschiebbar ist. Zur dynamischen Modulation der Divergenz 8 des Laserstrahls L ist die bewegliche Linse 151 mit einem Bewegungstreiber gekoppelt. Wie im Beispiel der Figur 3 ersichtlich ist, weist der Laserstrahl L bei einer ersten Grundstellung 151' der beweglichen Linse eine entsprechende Divergenz δ₁ auf. Bei einer Verschiebung der beweglichen Linse 151 auf der Übertragungsachse w verändert sich die Divergenz 8 des Laserstrahls L kontinuierlich und hat bei der um die Auslenkungsdistanz Δ verschobenen Stellung 151" eine veränderte Divergenz δ₂. Der Laserstrahl L mit der vom Divergenzmodulator 15 modulierten Divergenz δ, δ₁, δ₂ wird in den Figuren 1b, 2b und 3 mit dem Bezugszeichen Ld bezeichnet.

In alternativen Ausführungen umfasst der Divergenzmodulator 15 einen räumlichen Lichtmodulator zum Modulieren der Wellenfront des Laserstrahls L, ein Flächenlichtmodulator zum Modulieren der Reflexionswinkel an mehreren Punkten einer Reflexionsfläche, über die der Laserstrahl L geleitet wird, ein Brechungsmodulator zum Modulieren des Brechungsindexes eines optischen Elements an mehreren Punkten im Querschnitt des Strahlengangs und/oder einen Amplitudenmodulator zur Amplitudenmodulation an mehreren Punkten im Querschnitt des Strahlengangs, also im Strahlprofil, des Laserstrahls L.

Der Divergenzmodulator 15 ist eingerichtet, die Divergenz δ, δ₁, δ₂ des Laserstrahls L (während der Scannbewegung s') mit mindestens gleich grosser Frequenz respektive Geschwindigkeit zu modulieren, mit der das Scannersystem 14 die Scannbewegung s' über den Scannwinkel β ausführt. Überdies ist der Divergenzmodulator 15 mit dem Scannersystem 14 so gekoppelt, dass die Veränderung der Divergenz δ, δ₁, δ₂ des Laserstrahls L mit dem Scannwinkel β, β₁, β₂ der Scannbewegung s' synchronisiert ist. Wie in der Figur 2b schematisch dargestellt ist, ergibt sich so eine sich mit dem Scannwinkel β, β₁, β₂ verändernde, d.h. vom Scannwinkel β, β₁, β₂ abhängige Divergenz δ, δ₁, δ₂ des Laserstrahls L.

Der Divergenzmodulator 15 ist hinsichtlich Modulationsfrequenz respektive Modulationsgeschwindigkeit und Modulationstiefe respektive Modulationsintensität, z.B. in der Ausführung nach Figur 3 die Auslenkungsdistanz Δ, vom Steuermodul 40 einstellbar und steuerbar. Die Modulationsfrequenz des Divergenzmodulators 15 ist beispielsweise mit der Scanngeschwindigkeit des Scannersystems 14 synchronisiert, z.B. wird während einer Scannbewegung s' über den Scannwinkel β (z.B. von β₁ auf β₂) eine Verschiebung über die Auslenkungsdistanz Δ durchgeführt.

Wie bereits oben im Zusammenhang mit dem optischen Element 13 beschrieben wurde, ergibt sich bei einer abhängig vom Scannwinkel β, β₁, β₂ veränderten Divergenz δ, δ₁, δ₂ des Laserstrahls L eine vom Scannwinkel β, β₁, β₂ abhängige Verschiebung des Fokus F, F* des projizierten Laserstrahls L* wie in der Figur 2b beispielhaft illustriert ist. Bei entsprechender Synchronisation der Veränderung der Divergenz δ, δ₁, δ₂ des Laserstrahls L mit dem Scannwinkel β, β₁, β₂ der Scannbewegung s' resultiert eine Verkippung der Scannlinie s. Der Verkippungswinkel γ zwischen der verkippten Scannlinie s* und der unverkippten Scannlinie s in der durch die optische Achse o der Projektionsoptik 10 und der Scannlinie s gebildeten Ebene ist durch die Modulationstiefe respektive Modulationsintensität einstellbar, z.B. durch die Auslenkungsdistanz Δ.

Wenn der Divergenzmodulator 15 eingerichtet ist, die Divergenz δ, δ₁, δ₂ des Laserstrahls L mit grösserer Frequenz respektive Geschwindigkeit zu modulieren, als das Scannersystem 14 die Scannbewegung s' ausführt, ermöglicht dies die Scannlinie s nicht bloss um den Verkippungswinkel γ zu verkippen, sondern die Scannlinie s in der durch die optische Achse o der Projektionsoptik 10 und der unverkippten Scannlinie s gebildeten Ebene zu verformen, wobei bei einer variierenden Modulationsgeschwindigkeit auch eine "nichtlineare Verkippung" und damit eine Verformung der Scannlinie s in der Projektionsrichtung ermöglicht wird.

In der Ausführungsvariante mit dem optionalen Scannersystem 11, welches das Augengewebe mit dem Laserstrahl L respektive den Laserpulsen entlang einer Bearbeitungslinie b scannt, der die Scannbewegung s' des vorgeschalteten Scannersystems 14 überlagert ist, ermöglicht der Divergenzmodulator 15 eine vom Scannwinkel β, β₁, β₂ abhängige Divergenz δ, δ₁, δ₂ des Laserstrahls L für eine gezielte Verkippung der durch die Scannlinie s und die Bearbeitungslinie b definierten Schneidfläche. Bei einer Modulationsgeschwindigkeit des Divergenzmodulators 15 die höher ist als die Scanngeschwindigkeit des Scannsystems 14 ermöglicht der Divergenzmodulator 15 eine gezielte Verformung dieser Schneidfläche.

Das Steuermodul 40 ist eingerichtet den Divergenzmodulator 15 so einzustellen, dass durch die vom Scannwinkel β, β₁, β₂ abhängige Divergenz δ, δ₁, δ₂ des Laserstrahls L eine Verkippung der Scannlinie s respektive der durch die Scannlinie s und die Bearbeitungslinie b definierten Schneidfläche um den vorgegebenen Verkippungswinkel γ bewirkt wird. Dazu umfasst das Steuermodul 40 eine Steuerfunktion, welche für vorgegebene respektive während der Bearbeitung des Augengewebes laufend neu berechnete Verkippungswinkel γ jeweils zugeordnete Steuerwerte zur Einstellung des Divergenzmodulators 15 bestimmt, insbesondere zur Einstellung der Modulationstiefe respektive Modulationsintensität, z.B. die Auslenkungsdistanz Δ, und der Modulationsgeschwindigkeit, wobei die Synchronisation zwischen dem Scannersystem 14 und dem Divergenzmodulator 15 vorzugsweise über gemeinsame Synchronisationsleitungen respektive Synchronisationssignale erfolgt. Für eine gezielte Verformung der durch die Scannlinie s und die Bearbeitungslinie b definierten Schneidfläche bei entsprechend grosser Modulationsgeschwindigkeit des Divergenzmodulators 15 steuert das Steuermodul 40 den Divergenzmodulator 15 mit dynamisch ändernden Steuerwerten für eine während der Scannbewegung s' variierende Modulationstiefe respektive Modulationsintensität, z.B. die Auslenkungsdistanz Δ, und/oder Modulationsgeschwindigkeit.

In einer Ausführungsvariante gemäss Figur 1b umfasst die ophthalmologische Vorrichtung 1 bloss ein Scannersystem 14 oder 11, beispielsweise ein Galvanoscannersystem, das zwischen den Divergenzmodulator 15 und die Projektionsoptik 10 geschaltet ist und das eingerichtet ist, das Augengewebe zweidimensional, also sowohl in einer ersten Abtastrichtung x und einer dazu senkrechten zweiten Abtastrichtung y (siehe Figur 4a) entlang einer Scannlinie s respektive Bearbeitungslinie b bearbeitend zu scannen (abzutasten). Das Steuermodul 40 ist eingerichtet, das Scannersystem 14 respektive 11 für die Bearbeitung des Augengewebes entlang verschiedener Scannlinien s respektive Bearbeitungslinien b zu steuern, die beispielsweise eine mäandrierende, spiralförmige, kreisförmige oder eine freie Form aufweisen und sich über einen Teil oder das gesamte zu bearbeitende Gebiet B des Augengewebes erstrecken. Das Steuermodul 40 ist zudem eingerichtet, die Bearbeitungstiefe in der zur ersten Abtastrichtung x und zweiten Abtastrichtung y senkrechten z-Richtung durch entsprechende Einstellung des Divergenzmodulators 15 zu steuern. Die Bearbeitungstiefe in z-Richtung ist somit durch entsprechende Ansteuerung des Divergenzmodulators 15 durch das Steuermodul 40 während der Bearbeitung des Augengewebes in den Abtastrichtungen x, y entlang der Scannlinie s respektive Bearbeitungslinie b modulierbar. Dadurch sind nicht nur ebene sondern auch gekrümmte Schneidflächen im Augengewebe möglich, insbesondere gezielt geformte Schneidflächen, beispielsweise Teile von Kugeloberflächen, Ellipsoidoberflächen, eindimensional oder zweidimensional gewellte Formen oder andere Freiformflächen, ohne dass dazu die Projektionsoptik 10 oder optische Komponenten davon bewegt werden müssen. Dabei sind sowohl der durch die veränderbare Einstellung der Modulationstiefe respektive Modulationsintensität des Divergenzmodulators 15 in z-Richtung ermöglichte Tiefenverstellungsbereich im Augengewebe als auch der Auslenkungsbereich der dafür erforderlichen Auslenkungsdistanz Δ der Linse 151 wesentlich kleiner als die Dicke der Cornea 22, der Linse oder anderer Gewebeteile des Auges. Somit ist im Augengewebe eine Modulation der Bearbeitungstiefe in z-Richtung mit einer wesentlich höheren Frequenz möglich als dies durch Bewegung der grossen Masse der Projektionsoptik 10 oder optischer Komponenten davon möglich wäre.

Die Figur 1c stellt eine ophthalmologische Vorrichtung 1 dar, in welcher das Verkippungssystem 4 auf der Projektionsoptik 10 basiert, die um eine zu ihrer optischen Achse o und zur Scannlinie s senkrecht verlaufenden Drehachse q (oder q') verkippbar ausgestaltet ist. Wie mit der Drehachse q' angedeutet ist, braucht die Drehachse q' nicht direkt durch die optische Achse o und die Scannlinie s zu verlaufen, es genügt, wenn die Drehachse q, q' senkrecht zu einer durch die Scannlinie s und die optische Achse o definierten Ebene verläuft.

Wie in der Figur 1d dargestellt ist, bewirkt eine Verkippung der Projektionsoptik 10 um die Drehachse q, q' mit dem Drehwinkel γ* eine Verkippung der Scannlinie s um den Verkippungswinkel γ=γ*. Im Beispiel der Figur 1d, ist die verkippte Scannlinie s* in der durch die Scannlinie s und die optische Achse der Projektionsoptik definierten Ebene gegenüber der unverkippten Scannlinie s um den selben Verkippungswinkel γ verkippt, wie die optische Achse o der Projektionsoptik 10 gegenüber einer Oberflächennormalen n zum Kontaktkörper 31. Der Verkippungswinkel γ und der Drehwinkel γ* können aber auch unterschiedlich sein, wenn z.B. die objekt- und beidseitigen Brechungsindizes der Projektionsoptik 10 unterschiedlich sind.

In der Ausführungsvariante mit dem optionalen Scannersystem 11, welches das Augengewebe mit dem Laserstrahl L respektive den Laserpulsen entlang einer Bearbeitungslinie b scannt, der die Scannbewegung s' des vorgeschalteten Scannersystems 14 überlagert ist, ermöglicht die um die Drehachse q, q' verkippbare Projektionsoptik 10 eine entsprechende Verkippung der durch die Scannlinie s und die Bearbeitungslinie b definierten Schneidfläche.

Zum Einstellen und Fixieren der Verkippung der Projektionsoptik 10 und der resultierenden Verkippung der Scannlinie s respektive Schneidfläche umfasst die ophthalmologische Vorrichtung 1 in einer Ausführungsvariante eine mit der Projektionsoptik 10 gekoppelte Justiervorrichtung 16.

Zur automatisierten Verkippung der Projektionsoptik 10 umfasst die ophthalmologische Vorrichtung 1 in einer weiteren Ausführungsvariante einen mit der Projektionsoptik 10 gekoppelten Antrieb 17. Überdies ist das Steuermodul 40 mit dem Antrieb 17 verbunden zur Steuerung der Verkippung der Projektionsoptik 10 und der daraus resultierenden Verkippung der Scannlinie s respektive der Schneidfläche gemäss einem vorgegebenen respektive während der Bearbeitung des Augengewebes laufend neu berechneten Verkippungswinkel γ.

## Patentansprüche

1. Ophthalmologische Vorrichtung (1) zum Bearbeiten von Augengewebe (22) mittels eines gepulsten Laserstrahls (L), umfassend:
eine Projektionsoptik (10) eingerichtet zur fokussierten Projektion des Laserstrahls (L) ins Augengewebe (22),
ein der Projektionsoptik (10) vorgelagertes Scannersystem (14) eingerichtet zum strahlablenkenden Scannen des Augengewebes (22) mit dem Laserstrahl (L) in einer Scannbewegung (s') entlang einer Scannlinie (s), und
ein dem Scannersystem (14) vorgelagerter Divergenzmodulator (15), der eingerichtet ist, die Divergenz (δ₁, δ₂) des Laserstrahls (L) dynamisch zu verändern, **dadurch gekennzeichnet,**
**dass** der Divergenzmodulator (15) zur Synchronisation von Scannbewegung (s') und Divergenz (δ₁, δ₂) des Laserstrahls (L) mit dem Scannersystem (14) gekoppelt ist, und
**dass** die ophthalmologische Vorrichtung (1) ein Steuermodul (40) umfasst, das eingerichtet ist, den Divergenzmodulator (15) derart zu steuern, dass die Divergenz (δ₁, δ₂) des Laserstrahls (L) so moduliert wird, dass die Scannlinie (s) mit einem vorgegebenen Verkippungswinkel (γ) verkippt wird.

2. Ophthalmologische Vorrichtung (1) nach Anspruch 1, **dadurch gekennzeichnet, dass** der Divergenzmodulator (15) eingerichtet ist, die Divergenz (δ₁, δ₂) des Laserstrahls (L) mit mindestens gleich grosser Frequenz zu modulieren, mit der das Scannersystem (14) die Scannbewegung (s') ausführt.

3. Ophthalmologische Vorrichtung (1) nach einem der Ansprüche 1 oder 2, **dadurch gekennzeichnet, dass** das Scannersystem (14) eingerichtet ist, die Scannbewegung (s') über einen Scannwinkel (β) auszuführen, und dass der Divergenzmodulator (15) eingerichtet ist, die Divergenz (δ₁, δ₂) des Laserstrahls (L) für eine vom Scannwinkel (β) abhängige Verschiebung der fokussierten Projektion in Projektionsrichtung zu modulieren.

4. Ophthalmologische Vorrichtung (1) nach einem der Ansprüche 1 bis 3, **dadurch gekennzeichnet, dass** der Divergenzmodulator (15) eingerichtet ist, die Divergenz (δ₁, δ₂) des Laserstrahls (L) für eine gezielte Verkippung der Scannlinie (s) zu modulieren.

5. Ophthalmologische Vorrichtung (1) nach einem der Ansprüche 1 bis 4, **dadurch gekennzeichnet, dass** der Divergenzmodulator (15) eingerichtet ist, die Divergenz (δ₁, δ₂) des Laserstrahls (L) für eine Verkippung der Scannlinie (s) mit einem definierten Verkippungswinkel (γ) in einer durch die Scannlinie (s) und eine optische Achse (o) der Projektionsoptik (10) definierten Ebene zu modulieren.

6. Ophthalmologische Vorrichtung (1) nach einem der Ansprüche 1 bis 5, **dadurch gekennzeichnet, dass** der Divergenzmodulator (15) eingerichtet ist, die Divergenz (δ₁, δ₂) des Laserstrahls (L) mit grösserer Frequenz zu modulieren, als das Scannersystem (14) die Scannbewegung (s') ausführt, und die Divergenz (δ₁, δ₂) des Laserstrahls (L) für eine gezielte Verformung der Scannlinie (s) zu modulieren.

7. Ophthalmologische Vorrichtung (1) nach einem der Ansprüche 1 bis 6, **gekennzeichnet durch** ein dem Scannersystem (14) nachgelagertes weiteres, zweites Scannersystem (11) zum Scannen des Augengewebes (22) mit dem Laserstrahl entlang einer Bearbeitungslinie (b), wobei die Scannbewegung (s') des vorgelagerten, ersten Scannersystems (14) der Bearbeitungslinie (b) überlagert ist, und wobei der Divergenzmodulator (15) eingerichtet ist, die Divergenz (δ₁, δ₂) des Laserstrahls (L) für eine gezielte Verkippung einer **durch** die Scannlinie (s) und die Bearbeitungslinie (b) definierten Schneidfläche zu modulieren.

8. Ophthalmologische Vorrichtung (1) nach einem der Ansprüche 1 bis 5, **dadurch gekennzeichnet, dass** dem Scannersystem (14) ein weiteres, zweites Scannersystem (11) zum Scannen des Augengewebes (22) mit dem Laserstrahl (L) entlang einer Bearbeitungslinie (b) nachgelagert ist, wobei die Scannbewegung (s') des vorgelagerten, ersten Scannersystems (14) der Bearbeitungslinie (b) überlagert ist, und dass der Divergenzmodulator (15) eingerichtet ist, die Divergenz (δ₁, δ₂) des Laserstrahls (L) mit grösserer Frequenz zu modulieren, als das erste Scannersystem (14) die Scannbewegung (s') ausführt, und die Divergenz (δ₁, δ₂) des Laserstrahls (L) für eine gezielte Verformung einer durch die Scannlinie (s) und die Bearbeitungslinie (b) definierten Schneidfläche zu modulieren.

9. Ophthalmologische Vorrichtung (1) nach einem der Ansprüche 7 oder 8, **dadurch gekennzeichnet, dass** das erste Scannersystem (14) eingerichtet ist, die Scannbewegung (s') mit einer wesentlich höheren Geschwindigkeit auszuführen, als das zweite Scannersystem (11) das Augengewebe (22) entlang der Bearbeitungslinie (b) scannt.

10. Ophthalmologische Vorrichtung (1) nach Anspruch 1, **dadurch gekennzeichnet, dass** das Steuermodul (40) eingerichtet ist, den Divergenzmodulator (15) derart zu steuern, dass die Divergenz (δ₁, δ₂) des Laserstrahls (L) so moduliert wird, dass die Scannlinie (s) in einer durch die Scannlinie (s) und eine optische Achse (o) der Projektionsoptik (10) definierten Ebene mit dem vorgegebenen Verkippungswinkel (γ) verkippt wird.

11. Ophthalmologische Vorrichtung (1) nach einem der Ansprüche 1 oder 10, **dadurch gekennzeichnet, dass** das Steuermodul (40) eingerichtet ist, während des Bearbeitens des Augengewebes (22) einen veränderten Verkippungswinkel (γ) zu bestimmen und den Divergenzmodulator (15) derart zu steuern, dass die Scannlinie (s) mit dem veränderten Verkippungswinkel (γ) verkippt wird.

12. Ophthalmologische Vorrichtung (1) nach einem der Ansprüche 1 bis 11, **dadurch gekennzeichnet, dass** der Divergenzmodulator (15) zwei seriell angeordnete optische Linsen (151, 152) umfasst, wobei mindestens eine der Linsen (151) zur Modulation der Divergenz (δ₁, δ₂) des Laserstrahls (L), auf einer optischen Übertragungsachse (w) verschiebbar, mit einem Bewegungstreiber gekoppelt ist.

13. Ophthalmologische Vorrichtung (1) nach einem der Ansprüche 1 bis 11, **dadurch gekennzeichnet, dass** der Divergenzmodulator (15) mindestens eines aus der folgenden Liste umfasst: ein räumlicher Lichtmodulator zum Modulieren der Wellenfront des Laserstrahls (L), ein Flächenlichtmodulator zum Modulieren der Reflexionswinkel an mehreren Punkten einer Reflexionsfläche, ein Brechungsmodulator zum Modulieren des Brechungsindexes eines optischen Elements an mehreren Punkten im Querschnitt des Strahlengangs, und ein Amplitudenmodulator zur Amplitudenmodulation an mehreren Punkten im Querschnitt des Strahlengangs des Laserstrahls (L).

14. Ophthalmologische Vorrichtung (1) nach einem der Ansprüche 1 bis 13, **dadurch gekennzeichnet, dass** die Projektionsoptik (10) mit einer Fokussiervorrichtung (19) zum Einstellen einer Fokustiefe (F) versehen ist.

## Claims

1. Ophthalmological device (1) for processing eye tissue (22) by means of a pulsed laser beam (L), comprising:
a projection optical unit (10) designed for the focused projection of the laser beam (L) into the eye tissue (22),
a scanner system (14) disposed upstream of the projection optical unit (10) and designed for the beam-deflecting scanning of the eye tissue (22) with the laser beam (L) in a scanning movement (s') along a scanning line (s), and
a divergence modulator (15) disposed upstream of the scanner system (14), which modulator is designed to dynamically vary the divergence (δ₁, δ₂) of the laser beam (L), **characterized**
**in that** the divergence modulator (15) is coupled to the scanner system (14) for the purpose of synchronizing the scanning movement (s') and the divergence (δ₁, δ₂) of the laser beam (L), and
**in that** the ophthalmological device (1) comprises a control module (40), which is designed to control the divergence modulator (15) in such a way that the divergence (δ₁, δ₂) of the laser beam (L) is modulated such that the scanning line (s) is tilted with a predefined tilting angle (γ).

2. Ophthalmological device (1) according to Claim 1, **characterized in that** the divergence modulator (15) is designed to modulate the divergence (δ₁, δ₂) of the laser beam (L) with a frequency of at least the same magnitude as the frequency with which the scanner system (14) performs the scanning movement (s').

3. Ophthalmological device (1) according to either of Claims 1 and 2, **characterized in that** the scanner system (14) is designed to perform the scanning movement (s') over a scanning angle (β), and **in that** the divergence modulator (15) is designed to modulate the divergence (δ₁, δ₂) of the laser beam (L) for a displacement of the focused projection in the projection direction, said displacement being dependent on the scanning angle (β).

4. Ophthalmological device (1) according to any of Claims 1 to 3, **characterized in that** the divergence modulator (15) is designed to modulate the divergence (δ₁, δ₂) of the laser beam (L) for a targeted tilting of the scanning line (s).

5. Ophthalmological device (1) according to any of Claims 1 to 4, **characterized in that** the divergence modulator (15) is designed to modulate the divergence (δ₁, δ₂) of the laser beam (L) for a tilting of the scanning line (s) with a defined tilting angle (γ) in a plane defined by the scanning line (s) and an optical axis (o) of the projection optical unit (10).

6. Ophthalmological device (1) according to any of Claims 1 to 5, **characterized in that** the divergence modulator (15) is designed to modulate the divergence (δ₁, δ₂) of the laser beam (L) with a greater frequency than the scanner system (14) performs the scanning movement (s'), and to modulate the divergence (δ₁, δ₂) of the laser beam (L) for a targeted deformation of the scanning line (s).

7. Ophthalmological device (1) according to any of Claims 1 to 6, **characterized by** a further, second scanner system (11) disposed downstream of the scanner system (14) and serving for scanning the eye tissue (22) with the laser beam along a processing line (b), wherein the scanning movement (s') of the upstream first scanner system (14) is superimposed on the processing line (b), and wherein the divergence modulator (15) is designed to modulate the divergence (δ₁, δ₂) of the laser beam (L) for a targeted tilting of a cutting area defined by the scanning line (s) and the processing line (b).

8. Ophthalmological device (1) according to any of Claims 1 to 5, **characterized in that** a further, second scanner system (11) for scanning the eye tissue (22) with the laser beam (L) along a processing line (b) is disposed downstream of the scanner system (14), wherein the scanning movement (s') of the upstream first scanner system (14) is superimposed on the processing line (b), and **in that** the divergence modulator (15) is designed to modulate the divergence (δ₁, δ₂) of the laser beam (L) with a greater frequency than the first scanner system (14) performs the scanning movement (s'), and to modulate the divergence (δ₁, δ₂) of the laser beam (L) for a targeted deformation of a cutting area defined by the scanning line (s) and the processing line (b).

9. Ophthalmological device (1) according to either of Claims 7 and 8, **characterized in that** the first scanner system (14) is designed to perform the scanning movement (s') with a significantly higher speed than the second scanner system (11) scans the eye tissue (22) along the processing line (b).

10. Ophthalmological device (1) according to Claim 1, **characterized in that** the control module (40) is designed to control the divergence modulator (15) in such a way that the divergence (δ₁, δ₂) of the laser beam (L) is modulated such that the scanning line (s) is tilted with the predefined tilting angle (γ) in a plane defined by the scanning line (s) and an optical axis (o) of the projection optical unit (10).

11. Ophthalmological device (1) according to either of Claims 1 and 10, **characterized in that** the control module (40) is designed, during the processing of the eye tissue (22), to determine a changed tilting angle (γ) and to control the divergence modulator (15) in such a way that the scanning line (s) is tilted with the changed tilting angle (γ).

12. Ophthalmological device (1) according to any of Claims 1 to 11, **characterized in that** the divergence modulator (15) comprises two serially arranged optical lenses (151, 152), wherein at least one of the lenses (151) is coupled to a movement driver for the purpose of modulating the divergence (δ₁, δ₂) of the laser beam (L), in a manner displaceable on an optical transmission axis (w).

13. Ophthalmological device (1) according to any of Claims 1 to 11, **characterized in that** the divergence modulator (15) comprises at least one from the following list: a spatial light modulator for modulating the wavefront of the laser beam (L), a surface light modulator for modulating the reflection angles at a plurality of points of a reflection surface, a refraction modulator for modulating the refractive index of an optical element at a plurality of points in the cross section of the beam path, and an amplitude modulator for amplitude modulation at a plurality of points in the cross section of the beam path of the laser beam (L).

14. Ophthalmological device (1) according to any of Claims 1 to 13, **characterized in that** the projection optical unit (10) is provided with a focusing device (19) for setting a depth of focus (F).

## Revendications

1. Dispositif ophtalmologique (1) destiné à traiter des tissus oculaires (22) au moyen d'un faisceau laser pulsé (L), comprenant :
une optique de projection (10) destinée à focaliser la projection du faisceau laser (L) dans le tissu oculaire (22),
un système de balayage (14) disposé en amont de l'optique de projection (10) conçu pour balayer par déviation du faisceau le tissu oculaire (22) au moyen du faisceau laser (L) selon un mouvement de balayage (s') effectué le long d'une ligne de balayage (s), et
un modulateur de divergence (15) disposé en amont du système de balayage (14) qui est conçu pour modifier dynamiquement la divergence (δ₁, δ₂) du faisceau laser (L), **caractérisé**
**en ce que** le modulateur de divergence (15) est couplé au système de balayage (14) pour synchroniser le mouvement de balayage (s') et la divergence (δ₁, δ₂) du faisceau laser (L), et
**en ce que** le dispositif ophtalmologique (1) comprend un module de commande (40) qui est conçu pour commander le modulateur de divergence (15) de manière à ce que la divergence (δ₁, δ₂) du faisceau laser (L) soit modulée de telle sorte que la ligne de balayage (s) soit inclinée d'un angle d'inclinaison prédéterminé ( ).

2. Dispositif ophtalmologique (1) selon la revendication 1, **caractérisé en ce que** le modulateur de divergence (1 5) est conçu pour moduler la divergence (δ₁, (δ₂) du faisceau laser (L) à une fréquence au moins aussi élevée que celle à laquelle le système de balayage (14) effectue le mouvement de balayage (s').

3. Dispositif ophtalmologique (1) selon l'une quelconque des revendications 1 ou 2, **caractérisé en ce que** le système de balayage (14) est conçu pour effectuer le mouvement de balayage (s') sur un angle de balayage ( ), et **en ce que** le modulateur de divergence (15) est conçu pour moduler la divergence (δ₁, δ₂) du faisceau laser (L) pour un décalage de la projection focalisée dans le dispositif de projection, qui dépend de l'angle de balayage ( ).

4. Dispositif ophtalmologique (1) selon l'une quelconque des revendications 1 à 3, **caractérisé en ce que** le modulateur de divergence (15) est conçu pour moduler la divergence (δ₁, δ₂) du faisceau laser (L) pour une inclinaison ciblée de la ligne de balayage (s).

5. Dispositif ophtalmologique (1) selon l'une quelconque des revendications 1 à 4, **caractérisé en ce que** le modulateur de divergence (15) est conçu pour moduler la divergence (δ₁, δ₂) du faisceau laser (L) pour une inclinaison de la ligne de balayage (s) d'un angle d'inclinaison défini ( ) dans un plan défini par la ligne de balayage (s) et un axe optique (o) de l'optique de projection (10).

6. Dispositif ophtalmologique (1) selon l'une quelconque des revendications 1 à 5, **caractérisé en ce que** le modulateur de divergence (15) est conçu pour moduler la divergence (δ₁, δ₂) du faisceau laser (L) à une fréquence supérieure à celle à laquelle le système de balayage (14) effectue le mouvement de balayage (s'), et pour moduler la divergence (δ₁, δ₂) du faisceau laser (L) pour une déformation ciblée de la ligne de balayage (s).

7. Dispositif ophtalmologique (1) selon l'une quelconque des revendications 1 à 6, **caractérisé par** un second système de balayage (11) supplémentaire disposé en aval du système de balayage (14), qui est destiné à balayer le tissu oculaire (22) au moyen du faisceau laser le long d'une ligne de traitement (b), dans lequel le mouvement de balayage (s') du premier système de balayage (14) disposé en amont est superposé à la ligne de traitement (b), et dans lequel le modulateur de divergence (15) est conçu pour moduler la divergence (δ₁, δ₂) du faisceau laser (L) pour une inclinaison ciblée d'une surface de coupe définie par la ligne de balayage (s) et la ligne de traitement (b).

8. Dispositif ophtalmologique (1) selon l'une quelconque des revendications 1 à 5, **caractérisé en ce qu'**un second système de balayage (11) supplémentaire est disposé en aval du système de balayage (14), destiné à balayer le tissu oculaire (22) au moyen du faisceau laser (L) le long d'une ligne de traitement (b), dans lequel le mouvement de balayage (s') du premier système de balayage (14) disposé en amont est superposé à la ligne de traitement (b), et **en ce que** le modulateur de divergence (15) est conçu pour moduler la divergence (δ₁, δ₂) du faisceau laser (L) à une fréquence plus élevée que celle à laquelle le premier système de balayage (14) effectue le mouvement de balayage (s'), et pour moduler la divergence (δ₁, δ₂) du faisceau laser (L) pour une déformation ciblée d'un plan de coupe défini par la ligne de balayage (s) et la ligne de traitement (b).

9. Dispositif ophtalmologique (1) selon l'une quelconque des revendications 7 ou 8, **caractérisé en ce que** le premier système de balayage (14) est conçu pour effectuer le mouvement de balayage (s') à une vitesse sensiblement supérieure à celle à laquelle le second système de balayage (11) balaye le tissu oculaire (22) le long de la ligne de traitement (b).

10. Dispositif ophtalmologique (1) selon la revendication 1, **caractérisé en ce que** le module de commande (40) est conçu pour commander le modulateur de divergence (15) de manière à ce que la divergence (δ₁, δ₂) du faisceau laser (L) soit modulée de telle sorte que la ligne de balayage (s) soit inclinée d'un angle d'inclinaison prédéterminé ( ) dans un plan défini par la ligne de balayage (s) et un axe optique (o) de l'optique de projection (10).

11. Dispositif ophtalmologique (1) selon l'une quelconque des revendications 1 ou 10, **caractérisé en ce que** le module de commande (40) est conçu pour déterminer, pendant le traitement du tissu oculaire (22), un angle d'inclinaison modifié ( ) et pour commander le modulate de divergence (15) de manière à ce que la ligne de balayage (s) soit inclinée de l'angle d'inclinaison modifié ( ).

12. Dispositif ophtalmologique (1) selon l'une quelconque des revendications 1 à 11, **caractérisé en ce que** le modulateur de divergence (15) comprend deux lentilles optiques (151, 152) disposées en série, dans lequel au moins l'une des lentilles (151) est couplée à un dispositif d'entraînement de manière à pouvoir être décalée sur un axe de transmission optique (w) pour moduler la divergence (δ₁, δ₂) du faisceau laser (L).

13. Dispositif ophtalmologique (1) selon l'une quelconque des revendications 1 à 11, **caractérisé en ce que** le modulateur de divergence (15) comprend au moins l'un des éléments de la liste suivante : un modulateur spatial de lumière destiné à moduler le front d'onde du faisceau laser (L), un modulateur surfacique de lumière destiné à moduler l'angle de réflexion en une pluralité de points d'une surface de réflexion, un modulateur de réfraction destiné à moduler l'indice de réfraction d'un élément optique en une pluralité de points dans la section transversale du chemin de faisceau, et un modulateur d'amplitude destiné à moduler l'amplitude en une pluralité de points dans la section transversale du chemin de faisceau du faisceau laser (I).

14. Dispositif ophtalmologique (1) selon l'une quelconque des revendications 1 à 13, **caractérisé en ce que** l'optique de projection (10) est munie d'un dispositif de focalisation (19) destiné à régler une profondeur de foyer (F).
